Europäisches Patentamt

**European Patent Office**

**Office européen des brevets**

(11) Publication number: **0 283 285**
**A2**

(12) # EUROPEAN PATENT APPLICATION

(21) Application number: **88302339.2**

(22) Date of filing: **17.03.88**

(51) Int. Cl.⁴: **G 01 N 21/78**
G 01 N 21/59, G 01 N 33/49,
G 01 N 33/52, C 12 Q 1/54

(30) Priority: **17.03.87 US 26664**

(43) Date of publication of application:
**21.09.88 Bulletin 88/38**

(84) Designated Contracting States:
**AT BE CH DE ES FR GB GR IT LI LU NL SE**

(71) Applicant: **DIAGNOSTIC SYSTEMS, INC.**
**454 South Anderson Road**
**Rock Hill South Carolina 29730 (US)**

(72) Inventor: **Neilson, Charles F.**
**715 Buckeye Terrace**
**Rock Hill South Carolina 29730 (US)**

**Miller, David C.**
**44 Lakeside Drive**
**Greenbelt Maryland 20770 (US)**

(74) Representative: **Warren, Keith Stanley et al**
**BARON & WARREN 18 South End Kensington**
**London W8 5BU (GB)**

(54) **Method and apparatus for monitoring analytes in fluids.**

(57) The invention includes a photometer (10) and a photometric test strip (31) for use with the photometer for monitoring the presence and amount of an analyte in a liquid sample. The photometer (10) includes a light source (25) and a photodetector (26) which form an optical path for detecting light transmitted through a test strip (31). The latter has a dry support and an indicator portion, both of which allow light of predetermined frequencies to be transmitted therethrough. The photometer (10) includes an electrical circuit (21) for generating a signal proportional to detected light and for generating an output signal representative of the presence and amount of the analyte in the sample of blood being monitored. In a preferred embodiment, the invention includes the use of a nonbibulous test strip (31) having a nonbibulous indicator portion thereon through which light is transmitted and detected following deposition of a blood sample thereon. The photometer may be calibrated to produce an output reading and resonse to the transmitted light which is representative of the milligram percentage of glucose in the blood being monitored.

_Fig.1_

_Fig.3_

Bundesdruckerei Berlin

**Description**

## METHOD AND APPARATUS FOR MONITORING ANALYTES IN FLUIDS

The present invention relates to the detection of the presence of and the determination of the amount of an analyte in samples of a fluid. The invention particularly relates to a novel method, test device and instrument for self-diagnosis and self-monitoring of specific analytes in body fluids.

One particular body fluid analyte illustrative of the need for self-monitoring and in accordance with which the present invention is particularly useful is glucose, which is often monitored in samples of blood. There exists a number of medical reasons for seeking to determine blood glucose levels, the foremost of which may be the monitoring of patients with diabetes.

Diabetic patients fall into several categories, but in all cases should desirably monitor, or have monitored, their blood glucose level as regularly as possible. Ideally, diabetic patients will also have means available for determining their blood glucose levels on their own.

Recently, self-monitoring of blood glucose has become an important feature in the care of diabetic patients. Such self-monitoring provides the means for frequent and accurate measurement of a person's blood glucose. These measurements are important in a number of diabetic circumstances, including pregnancy, unstable diabetic conditions, propensities toward severe ketosis or hypoglycemia, an unexpected lack of the usual warning symptoms for hypoglycemia, the use of portable insulin infusion devices or multiple daily injections, and other particular circumstances.

Various tests for determining the presence of sugars (the broader category into which glucose falls) in solutions have been available for some time. The oldest include the Tollens and Benedict tests for reducing sugars. As is known to those familiar with such tests, although relatively straight-forward to the practicing chemist, they involve enough "wet" chemistry to preclude their use in self-monitoring. Additionally, such tests can only measure aqueous solutions, rather than samples of whole blood.

With the growth of spectroscopy and spectroscopic analysis techniques over the past few decades, both general and specific photometric devices have been developed which can identify chemical compounds based on their interaction with a range of electromagnetic radiation, as well as photometers specifically tailored to identify the presence and concentration of a single analyte such as glucose.

As used herein, the term "spectroscopy" refers to the analytical technique of directing incident light at a designated sample, reading the light following its interaction with the sample, and then making a determination of the contents of the sample based upon some measured differences between the incident light and the detected light. Devices for accomplishing such analysis can be referred to as "photometers", "spectrophotometers", or "spectrometers". As used herein, the term "photometer" will refer to such devices.

As originally developed such devices have been, and remain, rather large with delicate and complicated light paths, optics capable of generating an entire spectrum of light within a given range (ultraviolet, infrared, microwave, etc.), hardware for holding liquid samples and for directing the desired frequencies of light through the samples, and often a reference "blank" which carried a solvent identical to that used in the sample in order to provide appropriate calibration. Needless to say, such devices were useful in the hands of skilled chemists, but they do not provide any practical self-monitoring technique for individual diabetic patients.

Accordingly, instead of photometry, one of the most basic developments which allow diabetics to monitor their blood glucose has been the "test strip." A test strip is a small strip, commonly formed of bibulous material--i.e. a material which tends to soak up or "drink" liquids--a portion of which carries some chemi-cals which change color in the presence of an analyte such as glucose. Theoretically, the user can compare the color which develops with a standard chart and thereby measure and monitor one's blood glucose level. Treated, laboratory-grade filter paper is a very typical test strip material.

In use, the patient adds a small, precise sample of blood or urine to the test strip, waits a predetermined amount of time during which the strip changes color, and then compares the color of the strip to the standard color chart at the end of the time period. Given the difference in color perception between individuals, the characteristic poor vision of some diabetics, the difficulty with properly measuring the sample of blood or urine, the problems in precisely timing the reaction, the continuously changing color of the test strip, variations in the test strip chemistry from batch to batch, and other inherent inaccuracies of such testing methods, the results are often unreliable. Accordingly, the development of simple photometers with which lay persons could theoretically read such strips on a more consistent basis has been desirable.

The development of "microelectronic" technology has made such photometers available, and where the sole purpose of the device is to determine the presence and amount of a single known analyte such as glucose, the design can be somewhat simplified. Accordingly, a number of smaller instruments have been developed which not only provide simplified testing in medical laboratories, but now have been developed to the point where they are marketed as self-monitoring devices with which lay persons can attempt to diagnose their own blood chemistry.

Although the concept of such self-monitoring and the potential availability of third party payment (e.g. Medicare) for such devices has made their use attractive and popular, there still remain a number of disadvantages in the cost, design, use, precision and accuracy of presently available devices.

A primary source of these present disadvantages is the fact that 40 percent of all diabetics are over the age of 65. As a result, many diabetics are elderly and have difficulty in operating test devices which are relatively complicated and require much user dexterity, even though such devices may be quite simplified compared to laboratory techniques. Research in this area has shown that the major source of variability in the results obtained with self-monitoring blood glucose devices is attributable to error on the part of the user. Accordingly, self-monitoring devices and techniques which give acceptable results under controlled conditions often fail to give adequate results in actual patient care situations. According to recent evaluations, the accuracy of the results obtained from all currently available self-monitoring systems are highly dependent upon user skills.

Additionally, because most diabetic patients are required to report their self-diagnosis reports to their doctors, there exists the temptation for patients to attempt to misuse the devices or misrepresent the results obtained in order to avoid "doctor's orders" as to changes in diet, medication, or lifestyle, even though such activity may be life threatening.

Presently, all compact photometers designed for use by lay persons operate on one similar principal with associated characteristic disadvantages: the reflectance technique. In most reflectance photometers light is directed onto an opaque test strip and is reflected therefrom onto a detector. The difference in intensity between the incident light and the reflected light is proportional to the presence and the amount of blood glucose present in a sample and can be used to produce a readable output.

In spite of the theoretical possibilities of reflectance-type photometric testing of diagnostic strips, the reality has been somewhat less positive, mainly because of limitations inherent in current test strips, available instruments and the reflectance technique itself. Reflectance measurement includes a number of inherent difficulties, the most characteristic of which is obtaining results which are both accurate (i.e. a true reading) and precise (i.e. an accurate result repeated consistently by the device). By their nature, reflectance measurements require a reflecting background either as part of or behind the sample, and as would be further expected, the nature of bibulous materials such as paper and fabric tends to continuously interfere with the accuracy and precision of reflectance measurements made thereupon.

In currently available devices, the reflectance measurement of blood glucose is based on the "rate method." The rate method follows and measures the progress of a chemical reaction in order to extrapolate back and determine the initial concentrations of the reacting chemicals. In theory, this is a very accurate method of determining the concentration of a material in a sample. In practice, however, especially in the field of compact, portable selfmonitoring devices intended for use by lay persons, the method raises several difficulties.

First, because many portable photometers cannot track a reaction from start to finish, they are calibrated on the basis of measuring a reaction that has proceeded for a precise amount of time; i.e. 30 seconds, 60 seconds, etc. The instrument "assumes" that the user has accurately timed the reaction on the test strip before placing the strip in the machine for measurement. As would be expected, improper timing by the user results in a measurement somewhat different from that for which the machine is calibrated and results in indications of blood chemistry level which are similarly inaccurate. Additionally, many currently available instruments require complex and sophisticated microprocessor based electronics to monitor the reflected signal, carry out intensive calculations, and convert the continuously changing rate measurements into output readings.

Another disadvantage of such systems arises from the presence of red blood cells in samples of whole blood. These cells tend to block both incident and reflected light and thus interfere in the measurement of analytes such as glucose in the plasma. Accordingly, most of these systems require some technique for removing red blood cells prior to making the reflectance measurement, while not destroying the sensitized portion of the test strip or removing all of the liquid sample being tested. Common techniques include blotting or wiping the bibulous material with a tissue or a customized wipe, rinsing the material with water or another solvent, covering the bibulous material with a small strip of removable material which filters the red blood cells, and several other methods. In short, removal of red blood cells from bibulous portions of test strips presents the user with a wide-ranging set of manipulative difficulties, any one of which may result in an erroneous result.

In addition to removing the red blood cells, some current home use reflectance photometers require the user to pause for two or three timed intervals during and between the strip, sample and instrument manipulative and measurement sequences. Therefore, as would be further expected, if the patient's timing of the reaction, or his manipulation of the wash or wipe sequence is in error, the calibration of the machine will be inappropriate for the strip and sample being tested and the readout will fail to provide the necessary, accurate result. Some studies indicate that where self-monitoring blood glucose devices are in general use, up to 50% of the values obtained vary more than 20% from correct reference values.

It is therefore an object of the present invention to provide a test strip and complimentary photometer, and method of use, which together form a monitoring system for determining the presence and amount of an analyte in a liquid sample. It is another object of the present invention to provide a method and apparatus for determining the presence and amount of an analyte in a liquid sample which avoids the disadvantages of reflectance measurement by using transmission photometric methods.

It is another object of the invention to provide a test strip which comprises a support and an indicator portion formed from a nonbibulous, homogenous, solidified deposit of reagents.

It is another object of the invention to provide a test strip with an indicator portion which is partially or totally transparent to light produced by the instrument for the determination of analyte levels using transmission photometry.

It is another object of the invention to provide a test strip with a reagent system which carries chemical indicators and precursors of which the behavior upon reaction is predictable and consistent.

It is another object of the invention to provide a compact, portable, hand-held photometer for reading diagnostic test strips inserted thereinto, which test strips are formed from a light transmissive support and a light transmissive indicator portion thereon.

It is another object of the invention to provide a diagnostic test strip to determine the presence and amount of an analyte in a sample of blood deposited thereon and which test strip includes an indicator portion transparent to light and a solid support likewise transparent to light.

It is another object of the invention to provide a nonbibulous solidified reagent test portion upon a nonbibulous support, which test portion remains in place upon the support during the normal washing and wiping operations commonly performed upon such strips.

It is another object of this invention to provide a method of measuring the extent of color formation at the end point of a chromophore related monitoring reaction rather than at intervals during the progress of such a reaction.

In order that the present invention may be more readily understood, reference will now be made, by way of example, to the accompanying drawings, in which:-

Figure 1 is a perspective view illustrating the photometer and test strip of the present invention;

Figure 2 is a perspective view of the rear of the photometer;

Figure 3 is a partially opened, partially exploded perspective view of the interior portions and optical block of the photometer;

Figure 4 is an enlarged, partial plan view of the front cover of the photometer showing a light bar and associated reference numerals;

Figure 5 is a top plan view of the upper portion of the optical block;

Figure 6 is a perspective view of the test strip of the present invention;

Figure 7 is another perspective view of the test strip of the present invention and illustrating a color change following deposition of a sample thereon;

Figure 8 is a schematic view of the light path of the instrument of the present invention and the relationship of the source, strip and detector thereto;

Figure 9 is a second schematic view of the light path; and

Figure 10 is a schematic diagram of the electrical circuit of the present invention.

The present invention comprises a combination of a portable, hand held photometer and a test device for insertion thereinto which together form a monitoring system for determining the presence and amount of an analyte in a liquid sample. The combination comprises a light source for directing light and for defining an optical path in the photometer and a test strip formed from a light transmissive solid support and a light transmissive indicator portion thereon. The strip receives light from the light source, and the indicator portion changes its light transmission characteristics upon deposition thereon of a liquid sample which includes the analyte. The indicator portion transmits light at least after deposition of a liquid sample thereon. The photometer includes means for positioning the indicator portion of the strip in the optical path, and means for generating an electric signal proportional to incident light transmitted through the indicator portion. Means in the instrument generate an output signal in response to the electric signal, the output signal being representative of the presence and amount of the analyte in the sample of blood being monitored. The invention also includes a method of diagnosing the presence of an analyte in a liquid sample, the diagnostic test strip, and the compact photometer.

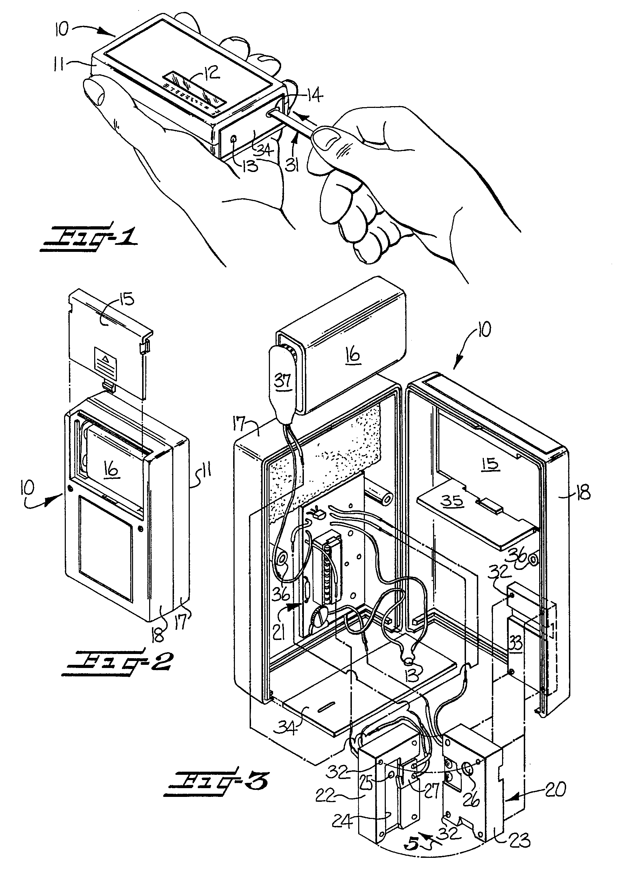

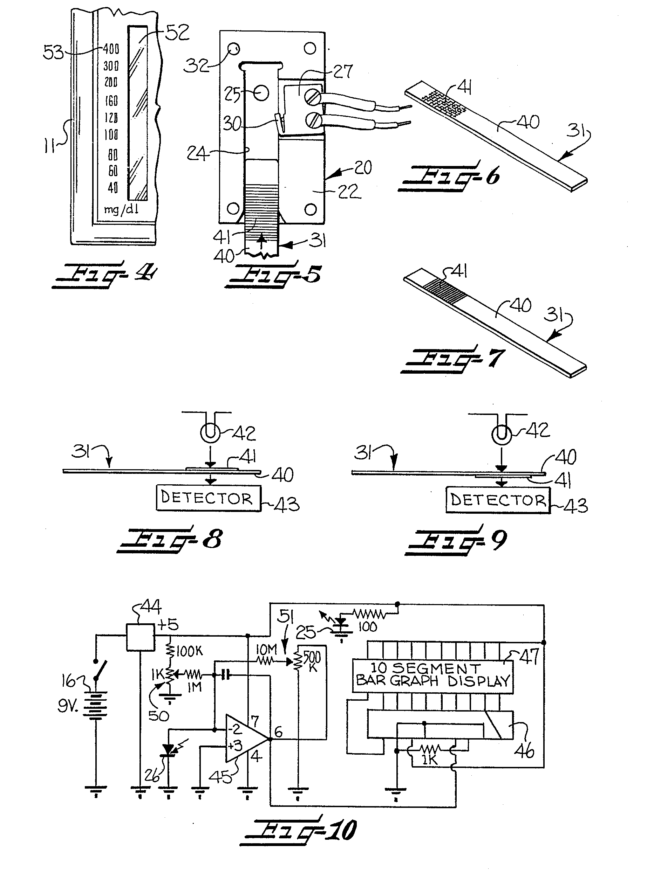

The present invention is a monitoring system comprising a novel instrument, a novel strip which the instrument is designed to read, a novel method of forming the strip and a method for using the strip and the instrument. As seen in Figures 1 and 2, the instrument is broadly designated at 10 and comprises a compact, portable, hand-held photometer for reading diagnostic test strips inserted thereinto. Figures 1 and 2 show an overall perspective view of the instrument 10 including the housing 11, the output display 12, an on/off indicator light 13 and the strip receiving opening 14. The bottom perspective view of Figure 10 also shows the battery cover 15 and the battery 16.

Figure 3 is a partially opened, partially exploded view of the photometer 10 and illustrates more of its particular components. These include the upper portion 17 and lower portion 18 of the housing 11, the electrical circuit board broadly designated at 21, and the battery 16.

The optical block is broadly designated at 20 and includes respective upper 22 and lower 23 portions which are illustrated in opened form in Figure 3 and show several other respective components to be described herein. As indicated in Figures 1 and 3, the optical block portions 22 and 23 are positioned immediately behind the strip receiving opening 14 and form a channel 24 which receives and positions a test strip in the instrument's optical path (Fig. 5).

In a preferred embodiment, the optical block's upper portion 22 includes a light-emitting diode 25 as the source and a light-sensitive diode 26 as the detector. Diodes 25 and 26 define an optical path therebetween within the photometer. Because the photometer can be tailored to the measurement of one particular analyte--glucose in this embodiment--the source diode 25 need not produce a wide range of frequencies, and intensities, but can be chosen to produce a selected frequency and intensity of light most appropriate to the diagnosis desired. In this respect, the output of the diode 25 and the chromophore in the reagent system can be tailored to complement one another to the greatest extent possible.

In this embodiment, the upper portion 22 of the light block also includes a microswitch 27 having a trigger portion 30 (Figure 5) which turns the instrument on when it is engaged by a strip 31 inserted thereinto. In Figure 5, the microswitch 27 is illustrated as being positioned approximately halfway along the channel 24. Because of the aforementioned tendency of some diabetic patients to attempt to misuse devices and so misrepresent their self-monitored glucose levels, however, the microswitch can alternatively be placed at the far-inward end of the channel 24 so that a strip must be completely inserted before the instrument 10 will operate. Using a photometer with an externally-operable switch or one with the microswitch located elsewhere, patients would have the tendency to attempt to trigger the device without inserting the entire test strip into the instrument and thereby obtain an inaccurate reading. As illustrated in Figures 3 and 5, the two portions 22 and 23 of the optical block can be fastened together and to the housing 11 by small screws (not illustrated) or in any other appropriate fashion. Passageways for such fasteners are shown at 32 in the upper and lower portions 22 and 23 of the optical block and in a block receiving portion 33 of the lower portion 20 of the housing 11.

As a further advantage, the transmission measurement techniques made possible by the present invention are generally much more forgiving of many types of misalignment of a test strip in relation to a source and detector than are reflectance techniques.

Figure 3 also illustrates a few of the structural features of the instrument 10. These include an end plate 34, a battery rack 35, fastener mounts 36, a battery terminal clip 37 and a number of electrical wires which, for the sake of clarity, are shown without specific reference numerals.

The invention also comprises a test device shown in the form of a strip 31 (Figs. 6 and 7) which can be read using transmission techniques. As mentioned earlier herein, all test strips otherwise marketed to date are intended for reflectance photometry and must include or be otherwise backed with some barrier for preventing the passage of light entirely through both the indicator portion and the support. In contrast, the test device 31 of the present invention has both a light transmissive solid support 40 and a light transmissive indicator portion 41 thereon. The indicator portion 41 receives light from the light source and comprises means for changing color upon deposition of a liquid sample including the designated analyte, such as glucose. Light can be transmitted through the indicator portion 41 and through the solid support 40 at least after deposition of the sample on the indicator portion.

The test strip 31 is straightforward in appearance, but its inherent characteristics are relatively sophisticated. In preferred embodiments of the device, the solid support 40 of the test strip 31 is nonbibulous and substantially transparent or transparent to light of the selected frequency or frequencies produced by the instrument's light source.

In a preferred embodiment of the invention and in contrast to many currently available test strips, the indicator portion 41 comprises a solidified deposit of reagents positioned directly upon the support 40. The solidified deposit comprises a single homogenous layer which is non-bibulous. As indicated earlier, the bibulous character of most other test strip indicator portions makes it impossible to use them in any transmission techniques.

Prior to the present invention, many of the attempts to produce strips incorporating such non-bibulous, solidified test portions depositied directly upon a solid support have been unsuccessful, especially where the solid support is also nonbibulous. It is difficult to adhere solidified deposits to the nonbibulous supports, and even where the deposits have initially adhered, they tend to become dislodged during test procedures, particularly the washing and wiping operations characteristic of self-monitoring blood glucose test strips used with currently available instruments.

It has been discovered according to the present invention, however, that a nonbibulous solidified reagent test portion can be successfully placed and maintained upon a nonbibulous solid support so as to remain deposited upon a test strip during normal testing procedures. Accordingly, the invention further comprises a method of preparing such a test strip, as will be described in further detail herein.

As background, many color-changing indicator systems for the detection of glucose operate according to the following sequence of reactions. In the first, glucose is allowed to oxidize to gluconic acid with a concurrent production of hydrogen peroxide. The reagent system of the present invention operates on this basis and includes an enzyme for catalyzing the oxidation of glucose. In the second, a chromophore (a molecule which has different colors in its oxidized and reduced states) oxidizes in the presence of the hydrogen peroxide produced during the oxidation of glucose. The present invention also includes an enzyme for catalyzing the oxidation of the chromophore in the presence of hydrogen peroxide.

A suitable chromophore will absorb light of the selected frequency produced by the source. In a preferred embodiment, the color-changing reagent system comprises the enzyme glucose oxidase, the enzyme horseradish peroxidase ("HRP"), 3-dimethylaminobenzoic acid, and 3-methyl-2benzothiazolinone hydrozone hydrochloride. A number of other chromophores are acceptable in the present invention, some of which include tetramethyl benzidine and ortho-tolidine and their respective derivatives.

The present invention, rather than being based on measurement of an ongoing reaction, can be based on measuring the extent of color formation at the end point of a reaction. Although measurement at an end point is theoretically less accurate than the aforementioned rate method, in practical terms its advantages for an individual lay user of instruments such as the photometer of the present invention are enormous.

First, the question of timing becomes much less critical. Once the reaction has reached an end point, the amount of chromophore developed will remain essentially the same and the user only has to wait at least a certain period of time (typically 30 seconds) after which the measurement will remain constant and accurate for a much longer period of time. In contrast, rate method photometers require that the reaction be measured within a small window of time or at one precise time interval measured from the start of the reaction.

In order to provide an end point reaction reagent system, the indicator portion of the test strips of the present invention use typical reagents in atypical amounts. Specifically, although most glucose test devices use an excess of HRP, the reagent system of the present invention limits the amount of HRP present. The nature of the reaction of HRP and the appropriate chromophores is such that the HRP both catalyzes the oxidation of the chromophore and is used up as part of the reaction. Accordingly, when the HRP is used up, the reaction will stop and the color change in the chromophore will remain constant.

With the color change constant and calibrated, this particular embodiment of the instrument eliminates the need for a microprocessor. This makes the machine smaller, lighter in weight, simpler, more reliable, and less expensive. In other circumstances, however, the inclusion and use of a microprocessor may be desirable and the invention is not limited to instruments which omit microprocessors.

In practice, the reagent system is essentially colorless and forms a rather neutral or white shade on the support portion 40 of the strip 31. Because many diabetic patients are older and because diabetes can affect vision, many users could have difficulty spotting the indicator portion 41 on the strip 31.

Accordingly, in preferred embodiments of the invention the diagnostic test strip includes an identifying colorant which distinguishes the indicator portion from the remainder of the strip prior to the deposition of a blood sample thereon. In this embodiment, a useful identifying colorant comprises tartrazine, which provides a visible yellow-green tint to the indicator portion (Fig. 5), but which does not interfere with the optical characteristics of the strip after deposition of a sample thereon.

In the present preferred embodiment, a test strip is prepared from alginates, adhesive, enzymes and chromophores. Its preparation proceeds as follows. First, a dilute solution of sodium alginate is prepared in deionized or distilled water. It has now been determined according to the present invention that the viscosity of the particular alginate used appears to be significant in producing a workable test strip. Alginates are natural products. They vary from source to source and can be selected according to their general viscosity. By selecting a higher viscosity alginate, a more stable solidified test portion results which successfully resists becoming dislodged during typical self-monitoring manipulations. Prior to the present invention, there appears to be no suggestion that the particular viscosity of an alginate could result in more stable solidified reagent deposits upon nonbibulous solid support strips.

Although some of the reasons for such success may remain presently unknown, it appears that less of a higher viscosity alginate is required to form an appropriate solid deposit resulting in a lower aqueous solubility of the deposit so formed. As stated earlier, washing operations typically use water as the wash solvent, and wiping operations attempt to wipe wet red blood cells from a wetted test portion of a strip. Such operations have little or no solubilizing and destabilizing effect on the less-soluble test portion of the strips of the present invention. In the remainder of the operation, the alginate solution is mixed with an emulsion of vinyl acetate and vinyl ethylene copolymer adhesive to form an alginate-adhesive emulsion. Glucose oxidase, HRP, and an appropriate chromophore are added to the emulsion and the resulting reagent mixture is placed upon appropriate portions of solid support material and allowed to dry into a solidified deposit. The solid support and solidified deposit can be formed or separated into strips of any desired size.

Example

A four percent solution of sodium alginate is prepared by adding 0.4g of the alginate to 10ml of deionized water. 0.2g of this solution are mixed with 0.8g of a polyvinyl acetate and polyvinyl ethylene copolymer adhesive in emulsion form. To this emulsion are added 0.12ml of glucose oxidase (approximately 1300 units total), 0.4mg of solid horseradish peroxidase (also approximately 1300 units total), 25mg of 3-dimethylaminobenzoic acid (DMAB), 3mg of 3-methyl-2-benzothiazolinone hydrozone hydrochloride (MBTH) and 150ug of tartrazine.

This material is folded until thoroughly mixed.

In order to form the solid support material, a calendar stock of plastic sheet is formed into longitudinal strips. A temporary channel is mechanically formed lengthwise along these strips, for example by using two parallel spaced-apart strips of cellophane adhesive, leaving a channel therebetween.

The emulsion mixture containing the reagents is applied to the channel and then, according to the particular mixture, either allowed to level, or mechanically leveled, e.g. by using a razor blade or some other leveling device, in order to get the proper thickness of the reagent mixture along the entire length of the channel.

The tape is then removed and hot air applied to set the mixture. Each longitudinal strip is then cut into widthwide strips in which the length is approximately the width of the original stock material. The resulting reagent portions are present on each of the resulting strips as a small rectangle or square.

The prepared strip and instrument are used together in the following manner. As schematically diagrammed in Figures 8 and 9, light travels along a path from the source 42 through the indicator portion 41, through the support portion 40 and to the detector 43. It will be understood that because Figures 8 and 9 are schematic illustrations, the indicator portion 41 is shown in exaggerated fashion as a rather significant and distinct layer on the strip 40 and not as the relatively thin coating that is actually formed and used in the practice of the invention. In the preferred embodiment, the detector may be either a photodiode or a photocell as desired. Because the light from the source passes entirely through both the indicator portion 41 and the support 40, the photometer and strip offer a significant fundamental advantage over prior devices: the strip can be inserted with the indicator portion facing either upwardly (Fig. 8) or downwardly (Fig. 9), and the instrument will still produce the same consistent, accurate result. In contrast, because of the nature of reflectance technology, the error of putting a strip in "upside down" results in no reading whatsoever. This advantage is fundamental because elderly or less dextrous persons need not concern themselves with overly precise positioning of the strip, and indeed, need not even take the trouble to orient the strip upwardly or downwardly. In essence, the instrument and the strip position themselves properly with respect to one another and require very little dexterity on the part of the user.

Figure 10 is a circuit diagram schematically illustrating the light source, electric signal generating means and output signal generating means of the present invention. In a preferred embodiment, the circuit includes the battery 16, the voltage regulator 44, the operational amplifier 45, a display driver 46 and the segmented bar graph display 47. The light source 25 comprises a silicon photocell. The detector 26 is a photodiode.

In operation, the source diode 25 produces light of a selected intensity and frequency which, as described earlier, passes through the indicator portion 41 of the strip 31 and falls incident upon the detector diode 26. The output of the detector diode 26 is passed to the operational amplifier 45 which in turn passes the amplified signal to the display driver 46.

In order to calibrate the output of the diode and amplifier to the capabilities of the bar graph display 47, the circuit includes two potentiometer circuits broadly designated at 50 and 51. Thus, the output of the nine-volt battery 16 is controlled by the voltage regulator 44 and the output of the detector diode 26 is calibrated on both the high voltage and low voltage ends of the display's 47 capability by the potentiometer circuits 50 and 51. The calibrated signal is then passed to the display driver 46 which produces an output on the bar graph display 47. The calibrated output corresponds to the amount of chromophore detected, which in turn reflects the amount of glucose in the sample, and proportionally represents the blood glucose level of the user providing the sample.

In the illustrated embodiment the photometer's display 47 responds to the calibrated voltage produced by the amplifier and the potentiometers. The display 47 includes ten segments (not individually illustrated), the first of which is activated by a potential of 0.13 millivolts, with each following segment being activated by an increase of 0.13 millivolts, so that a total of 1.3 millivolts lights all ten segments. The voltage regulator, potentiometer, amplifier, display driver and segmented display are all calibrated based on a predetermined relationship--which can easily be empirically developed--among the light produced by the source diode, the concentration of oxidized chromophore and the amount of light received by the photodetector.

In other embodiments, the display can comprise a digital display, typically a light emitting diode (LED) or liquid crystal display (LCD). The added precision provided by such displays is helpful to individuals who are monitoring certain analytes under certain circumstances. Where the analyte is blood glucose, for example, a digital display's more precise output is particularly helpful for type I diabetics.

It will be understood that the additional precision provided by a digital display is a function of the display's capabilities, and that the inherent advantages of the instrument and strip of the present invention are the same regardless of the type of output display selected.

Figure 4 illustrates the readout portion of the housing 11 and shows a longitudinal window 52 through which the output of the ten-segment bar graph display 47 may be viewed. A series of appropriate numerals 53 are included on the front of the housing 11, thereby providing the numerical indication required by the diabetic user.

In use, a test of blood glucose can be made as follows: first, the patient takes a drop of blood from a finger or other suitable body portion, usually using a small pin-like device produced and marketed for puncturing the skin for precisely this purpose. The drop of blood is next added to the indicator portion of the test strip. Because the strip is non-bibulous and because the reagent system is evenly distributed in a single homogenous layer which is deposited directly upon the support, the timing technique required of the user is minimal.

The user merely waits approximately 30 seconds for the reaction to take place after which the blood sample is simply wiped off of the indicator portion of the strip. Although it would appear that the entire blood sample is removed, in actuality the majority of the removed material represents red blood cells and the glucose-containing plasma is retained in the indicator portion. The strip changes color, the hue and shade of which depend upon the particular chromophore chosen and amount of glucose present, and which could be read using indicator charts as was commonly done using previous tests. The strip is then simply inserted into the instrument and the output noted. When the strip is properly inserted, the indicator light 13 turns on, signaling to the user that the machine is operating. With the window 52 of the ten-segment bar graph 47 appropriately labeled, the segment of the bar graph 47 which lights up is positioned next to the numerals 53 adjacent the window 52 which represents the amount of blood glucose present in the sample as detected by the chemistry of the strip and the circuitry of the instrument.

The precise amount of blood added to the test strip is not critical. Basically, the volume of glucose-containing blood plasma which can be absorbed by the indicator portion is relatively constant, regardless of the amount of blood added to the test strip. Accordingly, so long as the user makes sure that the indicator portion is covered with a sample of blood (usually one drop will suffice) the volume absorbed by the indicator portion will remain constant. Given a constant known quantity of blood plasma present in the sample, the intensity of chromophore development can easily be calibrated to the proportional amount of glucose present in the user's blood.

The typical glucose content of a normal person's blood will be on the order of 130 milligrams per deciliter (1/10 of a liter), a measurement which is often referred to diagnostically as "milligram percent." In the present embodiment, the photometer provides an output of milligram percentage levels between 40 and 400 milligram percent. It will be understood, however, that any appropriate range could be measured by the photometer of the invention by adjusting the corresponding calibration of the output.

In short, the invention provides a revolutionary simplicity in the accuracy of self-monitored diagnostic testing. The use of the strip is easier than almost all previous strips, the timing is simpler and less critical than most prior reactions required, and the instrument is extremely simple to use in comparison to previous devices.

Furthermore, it will be understood that although the particular embodiments of the instrument and the strips which have been described herein relate to the monitoring of blood glucose levels, any appropriate analyte can be similarly and suitably monitored according to the invention, and it will be further understood that the instrument, strip and techniques of the present invention are not limited to the field of medical diagnostics. There exist any number of industrial applications in which the presence, absence, or specific amounts of particular analytes in particular fluids should or must be monitored. In many of these, the availability of a straightforward test method and instrumentation with which persons other than laboratory chemists or technicians could monitor such analytes in such fluids, is highly desirable. As will be understood from the foregoing detailed description, the invention can provide such a method and instrumentation by incorporating an appropriate chromophore in the reagent systems in the test strips and a source and detector of corresponding sensitivity in the instrument.

In the drawings and specification, there have been disclosed typical preferred embodiments of the invention and, although specific terms are employed, they are used in a generic and descriptive sense only and not for purposes of limitation, the scope of the invention being set forth in the following claims.

**Claims**

1. A monitoring system for determining the presence and amount of an analyte in a liquid sample, the system comprising a photometer having a light source for generating a beam of light and for defining an optical path, a test strip positionable in the photometer, a photodetector for generating an electric signal proportional to incident light, and means for generating an output signal in response to the electric signal, characterized in that:

said test strip comprises a light transmissive, nonbibulous solid support and a nonbibulous indicator portion having alterable light transmission characteristics and comprising a solidified homogeneous deposit of a reagent system which changes light transmission characteristics upon deposition of a liquid sample including an analyte thereon; and

said photodetector and said light source are so arranged that the indicator portion of said test strip is positioned in said optical path so that light from said source is transmitted through said indicator portion and through said solid support to said detector to produce said electrical signal and said output signal, so that said output signal is representative of the presence and amount of an analyte in the liquid sample being monitored.

2. A monitoring system according to Claim 1 wherein said means for positioning said indicator portion of said strip comprises:

an optical block having respective upper and lower portions with a test strip receiving and positioning channel therebetween and with said light source being positioned adjacent said channel in one of said respective upper and lower portions and with said photodetector being positioned in said other respective portion and adjacent said channel and opposite from said source; and

means for operating said source, said detector and said signal generating means in response to a strip being properly positioned in said channel.

3. A monitoring system according to Claim 1 or 2 wherein said means for generating an output signal further comprises:

an operational amplifier electrically connected to said means for generating an electric signal;

a display driver electrically connected to said operational amplifier; and

a display responsive to said display driver for producing a visible signal representative of the presence and amount of analyte present in the liquid sample.

4. A monitoring system according to Claim 3 wherein said display responsive to said display driver comprises a segmented display.

5. A monitoring system according to Claim 3 wherein said display responsive to said display driver comprises a digital display.

6. A monitoring system according to any preceding Claim wherein said solidified deposit on said test strip changes color upon deposition of a liquid sample which includes glucose.

7. A monitoring system according to any preceding claim wherein said reagent system comprises:

an enzyme for catalyzing the oxidation of glucose to gluconic acid and hydrogen peroxide;

a chromophore which is oxidizable in the presence of hydrogen peroxide; and

an enzyme for catalyzing the oxidation of said chromophore in the presence of hydrogen peroxide.

8. A monitoring system according to Claim 7 wherein said reagent system further comprises an emulsion which includes a vinyl acetate and vinyl ethylene copolymer.

9. A monitoring system according to Claim 7 or 8 wherein said chromophore is selected from the group consisting of: 3-dimethylaminobenzoic acid, 3-methyl-2-benzothiazolinone hydrozone hydrochloride, tetramethyl benzidine and orthotolidine, and said enzyme for catalyzing the oxidation of glucose comprises horseradish peroxidose.

10. A diagnostic test strip for determining the presence and amount of an analyte in a liquid sample deposited thereon, said test strip comprising:

a light transmissive, nonbibulous solid support; and

a nonbibulous indicator portion deposited upon at least a portion of said support, said indicator portion comprising a solidified deposit of a reagent system which changes light transmission characteristics in the presence of the analyte, said indicator portion being transmissive at least to light of selected frequencies following addition of a liquid sample thereon, whereby the presence and amount of the analyte in a liquid sample deposited upon said strip may be determined by measuring the frequency and intensity of light transmitted through the liquid sample through the indicator system and through the solid support.

11. A diagnostic test strip according to Claim wherein said solidified deposit changes color in the presence of glucose.

12. A diagnostic test strip according to Claim 10 or 11 wherein said liquid sample comprises blood.

13. A diagnostic test strip according to Claim 10, 11 or 12 wherein said indicator portion further comprises a single homogeneous layer.

14. A diagnostic test strip according to any preceding Claim 10 to 13 wherein said reagent system comprises:

an enzyme for catalyzing the oxidation of glucose to gluconic acid and hydrogen peroxide;

a chromophore which is oxidizable in the presence of hydrogen peroxide; and

an enzyme for catalyzing the oxidation of said chromophore in the presence of hydrogen peroxide.

15. A diagnostic test strip according to Claim 14 wherein said chromophore is selected from the group consisting of 3-dimethylaminobenzoic acid, 3-methyl-2-benzothiazolinone hydrozone hydrochloride, tetramethyl benzidine and orthotolidine.

16. A diagnostic test strip according to any preceding Claim 10 to 15 wherein said solidified deposit of a reagent system comprises an alginate.

17. A compact, portable, hand held, battery operated photometer for reading test strips inserted thereinto which test strips are formed from a light transmissive support and a light transmissive indicator portion thereon comprising a reagent system for changing the light transmission characteristics thereof upon deposition of a liquid sample including an analyte thereon and for transmitting light at least after deposition of a liquid sample, said photometer comprising:

a light source for generating a beam of light of a selected frequency and intensity and for defining an optical path;

a photodetector for generating an electric signal proportional to incident light from said light source; and

means for generating an output signal in response to the electric signal;

characterized in that said photometer includes means for positioning the indicator portion of the test strip in said optical path so that light from said source passing along said optical path passes through the indicator portion and through the solid support and to the photodetector so that the output signal is representative of the presence and amount of an analyte in the liquid sample being monitored.

18. A compact photometer according to Claim 17 wherein said means for positioning the indicator portion of the strip comprises:

an optical block having respective upper and lower portions with a strip receiving and positioning channel therebetween and with said light source being positioned adjacent said channel in one of said respective upper and lower portions and with said photodetector being positioned in said other respective portion and adjacent said channel and opposite from said source.

19. A compact photometer according to Claim 18 further comprising means for operating said source, said detector and said signal generating means in response to a strip being properly positioned in said channel.

20. A method for monitoring the presence and amount of an analyte in a liquid sample, said method comprising:

(a) depositing a drop of liquid sample on a nonbibulous test strip which is transmissive at least to light of selected frequencies, and which test strip includes a light transmissive solid support and an indicator portion carrying a nonbibulous solidified homogeneous deposit of a chromophore reagent system which changes color in the presence of an analyte;

(b) placing the strip in a self-contained hand held photometer having a light source and a photodetector which together define an optical path, with the indicator portion of the strip being placed between the source and the photodetector and in the optical path;

(c) directing light from the source onto and through the indicator portion and the solid support and to the photodetector; and

(d) detecting the light transmitted through the indicator portion and through the solid support and to the photodetector and generating a signal responsive to the characteristics of the detected light.

21. A method according to Claim 20 wherein the step of depositing a drop of a liquid sample comprises depositing a drop of blood and removing the red blood cells from the test strip prior to the step of directing light onto the test strip.

22. A method according to Claim 20 or 21 wherein the chromophore reagent system changes color in the presence of glucose.

23. A method of preparing a test strip according to Claim 10 for monitoring analytes in small liquid samples, said method comprising:

forming a dilute aqueous solution of an emulsifying agent;

mixing the solution with an emulsified adhesive;

admixing a chromophore system which is sensitive to the presence of the analyte to be monitored with the mixture of adhesive and emulsifying agent;

applying a relatively thin layer of the adhesive, emulsifying agent and chromophore mixture to a support; and

drying the applied layer to thereby form a solidified, homogeneous layer of monitoring chemicals upon the support.

24. A method according to Claim 23 wherein the step of mixing the solution with an emulsified adhesive comprises mixing the solution with a vinyl acetate and vinyl ethylene copolymer.

25. A method according to Claim 24 wherein the step of admixing a chromophore system comprises admixing a reagent system which includes an enzyme for catalyzing the reaction of glucose to gluconic acid and hydrogen peroxide, a chromophore oxidizable in the presence of hydrogen peroxide, and an enzyme for catalyzing the oxidation of the chromophore in the presence of hydrogen peroxide.

26. A method according to Claim 23, 24 or 25 wherein the emulsifying agent comprises an alginate.

0283285

*Fig-1*

*Fig-2*

*Fig-3*

Fig-4

Fig-5

Fig-6

Fig-7

Fig-8

Fig-9

Fig-10